# EUROPEAN PATENT SPECIFICATION

(11) **EP 4 295 757 B1**
(45) Date of publication and mention of the grant of the patent: **23.09.2026**
(21) Application number: 23209094.4
(22) Date of filing: 15.01.2019
(51) Int. Cl.: A61B 5/024, A61B 5/01

(54) **METHOD, APPARATUS, AND SYSTEM FOR DETECTING STAGES OF SLEEP OF PERSON**
VERFAHREN, VORRICHTUNG UND SYSTEM ZUR ERKENNUNG VON SCHLAFSTADIEN EINER PERSON
PROCÉDÉ, APPAREIL ET SYSTÈME DE DÉTECTION DE STADES DE SOMMEIL D'UNE PERSONNE

(30) Priority: 19.01.2018 FI 20185051
(43) Date of publication of application: 27.12.2023
(62) Document number(s) of the earlier application(s) in accordance with Art. 76 EPC: 19704849.9 / 3 740 125
(73) Proprietor: Night Train Oy, 90420 Oulu (FI)
(72) Inventor: Rytky, Juha, 90420 Oulu (FI); Myllyoja, Kimmo, 90520 Oulu (FI); Pylkkö, Heikki, 54800 Savitaipale (FI)
(74) Representative: LEITZINGER OY

(56) References cited:
- EP-A1- 3 199 197
- WO-A1-2016/135382
- CN-A- 104 095 615
- US-A1- 2005 190 065
- US-A1- 2013 245 465
- US-A1- 2014 088 378
- US-A1- 2015 190 086

## Description

The invention relates to monitoring sleep of a person, and in particular, to detecting stages of sleep of the person.

### BACKGROUND INFORMATION

Sleep of a human follows a circadian rhythm, i.e. a sleep-wakefulness cycle that is on average 24.09 ± 0.2 h (24 h 5 min ± 12 min) for women and 24.19 ± 0.2 h (24 h 11 min ± 12 min) for men, aged 18 to 74 years. The circadian rhythm is typically synchronized to the day-night cycle, i.e. the light-darkness cycle. During sleep within the circadian rhythm, a natural sleep cycle repeats itself at a constant interval reflecting the 1-to-2-hour ultradian basic rest-activity cycle. The natural sleep cycle comprises a plurality of stages that repeat during the sleep. These stages include REM (Rapid Eye Movement) sleep and non-REM (NREM) sleep, which can be further divided in to light sleep and deep sleep.

An adult person may be considered to need on average a minimum of 4 full sleep cycles that are guided by the human circadian system controlling and the sleep-wake cycle. Less sleep may result in various negative effects to wellbeing and health. In order to ensure sufficient amount of sleep for a person, it may be desirable to try to monitor the sleep and to estimate the progress of the person's natural sleep cycles.

Various attempts have been made to estimate the progress of the natural sleep cycles, based on the nocturnal movements of a person, for example. Examples of prior art solutions are presented in EP3199197 A1 and US2014/0088378A1. However, there is no scientific evidence that the movements correlate with the sleep stages within the sleep cycle. Therefore, measurement of nocturnal movements of a person is not a reliable information source for tracking the sleep stages of the person. US-2013/245465-A1, US-2005/190065-A1, CN-104095615-A and US-2015/190086-A1 and WO 2016/135382 A1 describe further known methods for sleep stage evaluation based on temperature and heart rate measurements.

### BRIEF DISCLOSURE

An object of the present disclosure is to provide a method and an apparatus for implementing the method so as to alleviate the above disadvantages. The object of the disclosure is achieved by a method and apparatus which are characterized by what is stated in the independent claims. The preferred embodiments of the disclosure are disclosed in the dependent claims.

The present disclosure describes a method for detecting stages of sleep of a person. During REM (Rapid Eye Movement) sleep stage and deep sleep stage of a person's sleep, the person's body temperature control effectively shuts off and distal skin temperature changes on the limbs may be within just about 0,2 degrees Celsius. During the other sleep stages the human's temperature control is active and the distal skin temperature changes are much larger.

These two stages of sleep are therefore distinguishable from other stages of sleep and arousal (i.e. state of being awoken) of the person. However, it is difficult to distinguish these two stages from each other based on the temperature.

Heart rate variability (HRV) is another parameter that is affected by different stages of sleep. Importantly, REM sleep and deep sleep show different HRV characteristics. Therefore, it is possible to use HRV for distinguishing REM sleep and deep sleep from each other. By combining these two measurements (temperature variability and HRV), different stages of sleep can be identified.

The method according to the present disclosure provides accurate and cost-efficient means for detecting different stages of sleep. The measurements can be performed in a non-invasive manner that is more comfortable to the user. The method can be used for obtaining important information (e.g. ratios of different stages of sleep, length of sleep cycle, and circadian rhythm) on the sleep of the user.

### BRIEF DESCRIPTION OF THE DRAWINGS

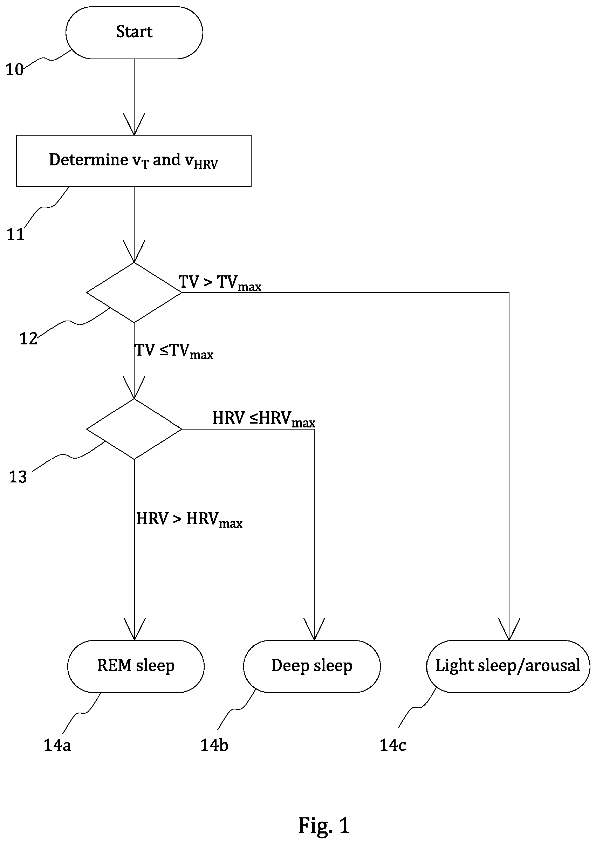

In the following the invention will be described in greater detail by means of preferred embodiments with reference to the attached drawings, in which
Figure 1 shows a simplified diagram of an exemplary embodiment of the method according to the present disclosure.

### DETAILED DISCLOSURE

The present disclosure describes a method for detecting stages of sleep of a person. A method according to the present disclosure comprises receiving temperature data and heart rate data of the person, determining temperature variability on the basis of the temperature data, determining heart rate variability (HRV) on the basis of the heart rate data, and detecting stages of sleep of the person on the basis of the temperature variability and heart rate variability. In the context of the present disclosure, the term "variability" represents dispersion (scatter, spread) of the measurements over time. The variability may be calculated as variance, standard deviation, and interquartile range of the measurements, for example.

The method may detect that the person is in a REM (Rapid Eye Movement) sleep stage when the temperature variability is below a first temperature variability limit and the heart rate variability is above a first heart rate variability limit. In other words, the method may comprise comparing the temperature variability with the first temperature variability limit, comparing the heart rate variability with the first heart rate variability limit, and considering that the person is in the REM sleep stage when a first condition is detected, the first condition being that the temperature variability is below the first temperature variability limit and the heart rate variability is above the first heart rate variability limit.

In addition or alternatively, the method may detect that the person is in a deep sleep stage when the temperature variability is below a second temperature variability limit and the heart rate variability is below a second heart rate variability limit. In other words, the method may comprise comparing the temperature variability with the second temperature variability limit, comparing the heart rate variability with the second heart rate variability limit, and considering that the person is in the deep sleep stage when a second condition is detected, the second condition being that the temperature variability is below the second temperature variability limit and the heart rate variability is below the second heart rate variability limit.

Figure 1 shows a simplified diagram of an exemplary embodiment of the method according to the present disclosure. In Figure 1, the first temperature variability limit is the same as the second temperature variability limit, and the first heart rate variability limit is the same as the second variability limit.

The method in Figure 1 starts from step initial step 10 from which the method moves to step 11. In step 11, the temperature variability TV and the heart rate variability HRV are determined and the method moves to step 12.

In step 12, the temperature variability TV is compared with a predetermined temperature variability limit TVₘₐₓ. If temperature variability TV exceeds the temperature variability limit TVₘₐₓ, the stage of sleep is considered to be light sleep stage or arousal and the method continues to a final step 14c identifying the sleep stage as light sleep or arousal. However, if temperature variability TV the same or less than temperature variability limit TVₘₐₓ, the method moves to step 13.

In step 13, the heart rate variability HRV is compared with a predetermined heart rate variability limit HRVₘₐₓ. If heart rate variability HRV exceeds the heart rate variability limit HRVₘₐₓ, the stage of sleep is considered to be REM sleep and the method continues to a final step 14a identifying the sleep stage as REM sleep. However, if heart rate variability HRV the same or less than the heart rate variability limit HRVₘₐₓ, the stage of sleep is considered to be REM sleep and the method continues to a final step 14b identifying the sleep stage as deep sleep.

A person has typically several brief periods of arousals during his/her nightly sleep. During these periods, the person is typically more restless, which can be detected as more physical activity. Therefore, in some embodiment, the method may further comprise measurement of physical activity (i.e. actigraphy). The measurement of physical activity of the person may be estimated based on an acceleration sensor attached to the person, for example. The measurement of physical activity can then be used for distinguishing the periods of arousal from the cycles of sleep. For example, in step 14c of Figure 1, arousal may be distinguished from light sleep based on the physical activity. The level of physical activity may be is compared with a predetermined physical activity limit, and if the level exceeds the limit, the stage of sleep may be considered to be arousal. If the level of physical activity does not exceed the limit, the stage of sleep may be considered to be light sleep.

Although Figure 1 uses only one limit for temperature variability and one limit for heart rate variability, the method according to the present disclosure may also utilize different temperature variability limits for REM sleep, deep sleep and light sleep/arousal. Alternatively or in addition, the method according to the present disclosure may also utilize different heart rate variability limits for REM sleep, deep sleep and light sleep/arousal.

With the method according to the present disclosure, important information on the quality of the sleep of a person can be obtained. Based on the detected stages of sleep, estimates of ratios of different stages of sleep during night can be calculated, for example. The ratios may be represented as percentages or amount (e.g. in minutes) of total sleep during the night, for example.

Another important parameter of sleep of a person is the interval (i.e. length) of a natural sleep cycle of the person. Typically, the interval (i.e. length) of a natural sleep cycle is approximately 90 minutes for an adult, but it may vary between individuals. For example, depending on the age of the person, the length of the sleep cycle may vary between 40 minutes to 130 minutes. However, within a time frame of a day or several days, the interval at which the natural sleep cycle repeats itself may be considered to remain rather constant. An adult person may be considered to need 4 to 6 full sleep cycles.

With the method according to the present disclosure, the interval between the occurrences of (or starts of) sleep cycles of specific type (e.g. REM sleep cycles or deep sleep cycles) can be detected. By determining the interval between these the sleep cycles, an estimate of the length of the natural sleep cycle of the person can be calculated. For example, a good estimate of the length of the sleep cycle may be calculated on the basis of an interval between the starts of the second and third REM sleep cycle after beginning of sleep. The estimate of the length of the sleep cycle may be based on an average of intervals between subsequent sleep cycles of the same type, for example.

Information on the length and contents of the natural sleep cycle can be coupled with the circadian rhythm, and in particular with melatonin onset (start of nocturnal melatonin production). There is a direct link between the melatonin onset and the circadian rhythm. The ability to fall asleep is linked to the melatonin onset. Thus, the melatonin onset provides a fixed reference point within the circadian rhythm of the person. The melatonin onset is observable as a change in the body temperature of the person. An estimate of the melatonin onset can be detected with a distal skin temperature measurement and an actigraphy measurement, for example. At the beginning of sleep of a person, his/her distal skin temperature rises rapidly for a while. However, at the same time the person typically remains relatively still which can be detected as a low actigraph value, e.g. in the form of low value (e.g. a value below a predetermined limit) of measurement data from an acceleration sensor.

Within the context of the present disclosure, temperature of a person may be measured in various ways. For example, the temperature samples may originate from a temperature sensor arranged to measure the distal skin temperature. In this context, distal skin temperature refers to skin temperature at extremities of body, such as the limbs and the head of a person. For example, distal skin temperature may be measured from a finger, wrist, ankle, forehead or ear lobe. The distal skin temperature may be measured with a MEMS-based temperature sensor or an infrared sensor, for example. In addition, the above-discussed phenomenon (i.e. change in the temperature variability) is detectable also in the core body temperature of the person albeit in smaller magnitudes. However, optical heart rate monitoring (e.g. photoplethysmography (PPG)) may be used to determine an estimate of relative core body temperature of the person. Further, an estimate of the heart rate may be extracted from graphical data provided by a digital video camera recording video of a portion of person's skin. Based on this estimate, variability of the relative temperature may be calculated, and changes in the variability of the relative temperature may then be used to detect the different stages of sleep and the melatonin onset.

The temperature sensor may be a part of a wearable device, for example. The temperature sensor may also be integrated to a vehicle, e.g. in the form of a video camera recording a view on the face of the driver of a car, and the method according to the present disclosure may be used for estimating the driver's alertness.

In order to be able to detect changes in the variability of the temperature, the temperature has to be sampled at a sufficient frequency. For example, the distal temperature may be sampled at least once a minute, e.g. once every 30 seconds. The temperature may be periodically sampled and the samples may be stored. Samples may be stored for one night or over a longer period, ranging from a plurality of days to a plurality of weeks, for example. Based on the samples, variability of the temperature may then be calculated by using known algorithms, for example. In case of longer measurement periods and larger quantities of data, the data may be sent from a wearable device making the measurements to a larger data storage, such as a computer server or a computing cloud, for example.

Information on detected stages of sleep may be utilized in various ways. For example, in a first embodiment of a method according to the present disclosure, the method comprises calculating ratios of different stages of sleep of the person based on the basis of the detected stages of sleep. This kind of information can provide an important insight to the quality of the sleep. The quality of sleep can have a significant effect on the wellbeing and health of a person. Thus, the method may be configured to provide information of ratios of different stages of sleep to the user. This information may then be utilized in estimating the quality of the sleep of the user, for example.

The detected stages may also be used for determining an optimal time window for sleep. In a second embodiment of a method according to the present disclosure, the method may comprise estimating an optimal instant for a wake-up for a person on the basis of the detected stages of sleep. If a wake-up (e.g. in the form of an alarm) is scheduled to occur at a light sleep stage within the natural sleep cycle, the person wakes up feeling fresh and energetic. Calculation of an optimal wake-up instant may comprise determining the length of a sleep cycle of the person, calculating the number of specific sleep stages already observed during night's sleep on the basis of the detected stages of sleep, and determining optimal wake-up instant on the basis of the length of the sleep cycle and the number of sleep stages already observed. For example, one or more lengths of the sleep cycle may be added to the last observed sleep stage of a specific type in order to determine an optimal wake-up instant so that the person will have slept a desired number (e.g. 4, 5, or 6) of sleep cycles at the optimal wake-up instant. Once the optimal instant has been reached, an alarm may be raised in order to wake up the person. In embodiments where the physical activity of the person is measured, an increased physical activity may be used as an indication of waking up from the sleep. For example, a detected increase the physical activity that is above a predetermined detection limit for a set amount of time may be used as an indicator that the alarm has performed its task.

In addition or alternatively in the second embodiment, estimates of optimal instants of wake-up may also be calculated beforehand on the basis of the length of the natural sleep cycle and a reference point within the circadian rhythm of the person, such as the melatonin onset. Thus, the method may comprise determining a length of a sleep cycle of the person, determining a fixed reference point within a circadian rhythm of the person, and determining the optimal wake-up instant on the basis of the length of the sleep cycle and the fixed reference point so that the person will have slept a desired number of sleep cycles at the optimal wake-up instant. For example, an optimal wake-up instant may be calculated by adding a plurality (e.g. 4, 5, or 6) of determined lengths of the natural sleep cycle to the time of a known time instant of the melatonin onset. Once the optimal instant has been reached, an alarm may be raised in order to wake up the person.

In addition or alternatively, the method according to the present disclosure may also be used for determining an estimate of an optimal instant for turning in for sleep. According to a third embodiment of the method according to the present disclosure, the method may determine the optimal sleep onset (i.e. an optimal instant for turning in for sleep) based on a reference point within the circadian rhythm of the person. The reference point may represent the melatonin onset, for example. The method may calculate the next optimal instant for a sleep onset based on the length of the sleep cycle, in case the person misses any preceding optimal instants for sleep onset.

In embodiments where distal skin temperature is measured, detection of a specific temperature change pattern within distal skin temperature data may be utilized in detecting the melatonin onset. A rapid, distinct pattern in the distal skin temperature can be observed after the nocturnal melatonin production has started. The temperature change pattern that is in the form of a drop in the distal skin temperature followed an increase in the distal skin temperature, wherein the drop and the increase occur within a time window of ten minutes or less, for example five minutes. The magnitude of the increase is higher than the magnitude of the drop. The magnitude of the drop may be approximately 0.5 °C and the magnitude of the increase may be approximately 1.5 °C, for example. When the characteristic temperature change pattern is observed in the distal temperature of a person, he or she has approximately 20 to 30 minutes to get to sleep. Thus, the temperature change pattern serves as a fixed reference point within the circadian rhythm. Once a person has fallen asleep, the temperature change patterns cease to occur. The temperature change patterns will re-start again if the person awakes in the middle of the night. This may be used as a further indicator of sleep when monitoring the sleep of a person. When she or he falls asleep, the distal skin temperature starts to increase constantly by about 2 °C in the forthcoming 120 minutes. By determining the interval between the occurrences of the temperature change patterns, another estimate of the length of the natural sleep cycle of the person can be calculated.

This additional information may be used for providing additional/alternative estimates on the circadian rhythm, the length of the sleep cycle, and the stages of the natural sleep cycle during the circadian rhythm. Certainty and accuracy of a detection method according to the present disclosure may be improved by combining the detection of stages of sleep and the detection of the temperature patterns. Alternatively, in some embodiments, the length of the sleep cycle may be based solely on the temperature change patterns. For example, the length of sleep cycle in the second embodiment discussed above may be based on the interval between temperature change patterns instead of the intervals between the detected sleep stages. The length of sleep cycle may also be based on the interval between temperature change patterns in the third embodiment above.

The accuracy of the estimate of the circadian rhythm and the length of the sleep cycle may be further improved by gathering data for a plurality of days and by using this data for calculating the estimate. In some embodiments, a method according to the present disclosure may further comprise monitoring movements of the monitored person in order to improve the accuracy of the determination of the sleep cycle and the circadian rhythm.

The present disclosure further discloses a detection unit and a system for detecting stages of sleep of a person. The detection unit may comprise means configured to receive samples of temperature and heart rate of the person, and carry out the steps of an above-described method according to the present disclosure. The detection unit may be a stand-alone apparatus or it may be a part of a larger system.

For example, the detection unit may be a stand-alone apparatus that comprises both sensors for measuring a distal skin temperature and heart rate of a person, and means for implementing the method for detecting stages of sleep of a person according to the present disclosure. The apparatus may comprise control unit comprising a computing device (such as a processor, an FPGA, or an ASIC) and a memory which may act as the means for implementing the method. The stand-alone apparatus may be in the form a wearable device, such as an activity bracelet or a heart rate monitor, that comprises a temperature sensor for monitoring a distal skin temperature of the person. The control unit in the wearable device may be configured to receive samples of the measured temperature (e.g. distal skin temperature) and heart rate from the sensors and detect the stages of sleep based on the samples. The control unit may be configured to calculate percentages or amount (e.g. in minutes) of different stages of sleep based on the sample, and the wearable device may comprise a display on which the calculated percentages may be shown.

The control unit of the wearable device may also be configured to estimate an optimal instant for a wake-up and/or an optimal instant for turning in for sleep on the basis of the detected stages of sleep. The wearable device may implement a timer or clock function, and once an optimal instant has been reached, the wearable device may cause an alarm to be raised, in the form of an audible or visual cue, for example. The wearable device may be configured to implement also other functionalities in addition to a method according to the present disclosure.

In some embodiments, the detection unit may also be a part of a larger detection system. For example, a detection system may comprise a separate measurement unit comprising a distal skin temperature sensor and a heart rate sensor and a separate detection unit that configured to receive samples of distal skin temperature and heart rate from the measurement unit. Alternatively or in addition, the measurement unit may provide the detection unit with temperature variability and/or heart rate variability data calculated on the basis of the distal skin temperature and heart rate data.

For simplicity, temperature data and temperature variability data may be jointly referred to as first data in the context of the present disclosure. Similarly, heart rate data and heart rate variability (HRV) data may be referred to as second data.

The measurement unit may comprise a wireless communication unit through which the temperature and heart rate data is sentto the separate detection unit. The wireless connection unit may transmit the temperature and heart rate data via Bluetooth, ZigBee, near field communication (NFC), or infrared protocols, for example. The measurement unit may be in the form of an activity tracker, a smartwatch, an earbud, a ring, an e-sticker (i.e. an adhesive sensor), for example. The separate detection unit may be a generic computing device or system, for example. A method according to the present disclosure may be implemented in the form of a computer program product having instructions which, when executed by the computing device or system, cause the computing device or system to perform a method according to the present disclosure, also including the first, second, and third embodiment described above. For example, the separate detection unit may be a handheld communication device, such as a smart phone or a tablet computer or even infotainment device or any kind of medical device, to which the computer program product is downloaded. The handheld communication device acting as the separate detection unit may be configured to wirelessly receive temperature and heart rate data and/or temperature variability and heart rate variability data originating from the measurement unit and detect and display sleep quality information, such as information on stages of sleep, on the basis of the data. The communications device may also determine an optimal time window for sleep of the person on the basis of the received data. The handheld communication device may be configured to estimate an optimal instant for a wake-up and/or an optimal instant for turning in for sleep on the basis of the determined optimal time window for sleep, and raise an alarm on the basis of the estimated optimal instant. The handheld-communication device may utilize personal preferences of the user stored in the device in the estimation of the optimal time window for sleep.

Alternatively, cloud computing may be utilized for implementing a method according to the present disclosure, also including the first, second, and third embodiment described above. For example, a cloud computing system acting as a separate detection unit may configured to receive temperature and heart rate data and/or temperature variability and heart rate variability data of a person, detect the stages of sleep and/or temperature change patterns on the basis of the data, and determine sleep quality information and/or an optimal time window for sleep of the person. The cloud computing system may be configured to estimate an optimal instant for a wake-up and/or an optimal instant for turning in for sleep on the basis of the determined optimal time window for sleep, and cause an alarm to be raised on the basis of the estimated optimal instant. For example, the cloud computing system may send an indication to a handheld communication device that an alarm should be raised. The handheld communication device then raises an alarm on the basis of this indication.

Functionalities of the method according to the present disclosure may also be divided between a cloud computing system and a separate decision-making unit. For example, the cloud may detect the stages of sleep and/or temperature change patterns as described above, and send information on the detected stages of sleep and/or temperature change patterns to a decision-making unit that estimates the quality of sleep and/or calculate and/or determines the optimal time window for sleep on the basis of the timing information. The decision-making unit may be a handheld communication device, for example. The decision-making unit may be further configured to calculate an optimal instant for a wake-up and/or an optimal instant for turning in for sleep, and raise an alarm on the basis of the estimated optimal instant based on personal preferences of the user stored on the decision-making unit.

The method (and a detection unit and system implementing the method) according to the present disclosure may be utilized in various applications. For example, in addition to the examples above, the method according to the present disclosure may also be utilized in minimizing jet lag. For a human, it is possible to adjust the circadian rhythm approximately by one hour per day. With the method according to the present disclosure, this adjustment can be done in a systematic manner.

In addition, since muscle recovery after exercise is closely related to the ratio of deep sleep within the total sleep, the information on ratios of different stages of sleep may be utilized when determining an optimal window of sleep for an athlete so s/he will have sufficient deep sleep for muscle recovery, for example. Further, humans need a sufficient time of REM sleep in order to be assimilate cognitive and motoric skills that are very essential skills e.g. for athletes. Recovery algorithms are based on the HRV and they can be significantly improved by using the quantity and quality of sleep. Further, medical personnel may utilize the information when determining the symptoms and cure of a patient in case of depression and sleep apnea, for example.

It is obvious to a person skilled in the art that the method and system according to the present disclosure can be implemented in various ways. The invention and its embodiments are not limited to the examples described above but may vary within the scope of the claims.

## Claims

1. A method for detecting stages of sleep of a person by a detection system comprising a computer implemented detection unit with a computing device or system, wherein the method comprises
receiving temperature data and heart rate data of the person from a measurement unit in the form of a wearable device comprising at least a distal skin temperature sensor and a heart rate sensor,
determining distal skin temperature variability of the person based on the temperature data overt time and determining heart rate variability (HRV) of the person based on the heart rate data,
detecting stages of sleep of the person within sleep of a person by
i) detecting that the person is in a Rapid Eye Movement (REM) sleep stage when the distal skin temperature variability is below a first distal skin temperature variability limit and the heart rate variability is above a first heart rate variability limit, and
ii) detecting that the person is in a deep sleep stage when the distal skin temperature variability is below a second distal skin temperature variability limit and the heart rate variability is below a second heart rate variability limit, and
providing information on quality of the sleep of the person based on the detected stages of sleep.

2. A method according to claim 1, wherein the method further comprises
estimating an optimal instant for a wake-up and/or an optimal instant for turning in for sleep based on the detected stages of sleep.

3. A method according to claim 2, wherein the method comprises
determining a length of a sleep cycle of the person, and
estimating the optimal wake-up instant based on the length of the sleep cycle so that, after a night's sleep, the person will have slept a desired number of sleep cycles at the optimal wake-up instant.

4. A method according to claim 2, wherein the method comprises
determining a length of a sleep cycle of the person,
determining a fixed reference point within a circadian rhythm of the person,
estimating the optimal wake-up instant based on the length of the sleep cycle and the fixed reference point so that the person will have slept a desired number of sleep cycles at the optimal wake-up instant.

5. A method according to any one of claims 2 to 4, wherein the estimating of the optimal instant for a wake-up or the optimal instant for turning in for sleep further comprises
gathering measurement data for a plurality of days,
calculating an estimate of the circadian rhythm of the person based on the gathered data, and
estimating the optimal instant for a wake-up and/or the optimal instant for turning in for sleep based on the detected stages of sleep and the estimate of the circadian rhythm.

6. A method according to any one of the preceding claims, wherein the providing of the information on quality of the sleep comprises
calculating ratios of different stages of sleep of the person based on the detected stages of sleep, and
using at least the calculated ratios as the information on the quality of sleep.

7. A detection system comprising a computer implemented detection unit with a computing device or system, wherein the detection system is configured to
receive first data and second data of the person from a measurement unit in the form of a wearable device comprising at least a distal skin temperature sensor and a heart rate sensor, wherein the first data represents distal temperature or distal temperature variability, and the second data represents heart rate or heart rate variability,
determine distal skin temperature variability of the person based on the temperature data over time and determining heart rate variability (HRV) of the person based on the heart rate data over time
detect the stages of sleep of the person within sleep of a person by
i) detecting that the person is in a Rapid Eye Movement (REM) sleep stage when the distal skin temperature variability is below a first distal skin temperature variability limit and the heart rate variability is above a first heart rate variability limit, and
ii) detecting that the person is in a deep sleep stage when the distal skin temperature variability is below a second distal skin temperature variability limit and the heart rate variability is below a second heart rate variability limit, and
provide the information on quality of the sleep of the person based on the detected stages of sleep.

8. A detection system according to claim 7, wherein the detection system is further configured to
estimate an optimal instant for a wake-up and/or an optimal instant for turning in for sleep based on the detected stages of sleep.

9. A detection system according to claim 8, wherein the estimating of the optimal instant for a wake-up or the optimal instant for turning in for sleep comprises
determining a length of a sleep cycle of the person, and
estimating the optimal wake-up instant based on the length of the sleep cycle so that, after a night's sleep, the person will have slept a desired number of sleep cycles at the optimal wake-up instant.

10. A detection system according to any one of claims 8 or 9, wherein the estimating of the optimal instant for a wake-up or the optimal instant for turning in for sleep further comprises
gathering measurement data for a plurality of days,
calculating an estimate of the circadian rhythm of the person based on the gathered data, and
estimating the optimal instant for a wake-up and/or the optimal instant for turning in for sleep based on the detected stages of sleep and the estimate of the circadian rhythm.

11. A detection system according to any one of claims 7 to 10, wherein the detection system comprises
a detection unit configured to perform the steps of receiving the first data and the second data, detecting the stages of sleep, and providing the information on quality of the sleep, wherein the detection unit is a handheld communication device configured to wirelessly receive the first data and the second data from a measurement unit.

12. A detection system according to claim 11, wherein the detection system further comprises a measurement unit in the form of a wearable device, such as a ring, an activity tracker, a smartwatch, an earbud, an e-sticker or a band, comprising at least:
a temperature sensor configured to measure distal skin temperature,
a heart rate sensor, and
a wireless communication unit, and
wherein the measurement unit is configured to determine the first data and the second data and send the first data and the second data to the detection unit through the wireless communication unit.

13. A computer program product having instructions which, when executed by a computing device, cause the computing device to
receive first data and second data of the person from a measurement unit in the form of a wearable device comprising at least a distal skin temperature sensor and a heart rate sensor, wherein the first data represents distal temperature or distal temperature variability, and the second data represents heart rate or heart rate variability (HRV),
detect the stages of sleep within sleep of a person by
i) detecting that the person is in a Rapid Eye Movement (REM) sleep stage when the distal skin temperature variability is below a first distal skin temperature variability limit and the heart rate variability is above a first heart rate variability limit, and
ii) detecting that the person is in a deep sleep stage when the distal skin temperature variability is below a second distal skin temperature variability limit and the heart rate variability is below a second heart rate variability, and
provide the information on quality of the sleep of the person based on the detected stages of sleep.

## Patentansprüche

1. Verfahren zum Erfassen von Schlafphasen einer Person durch ein Erfassungssystem, das eine computerimplementierte Erfassungseinheit mit einer Computervorrichtung oder einem Computersystem umfasst, wobei das Verfahren Folgendes umfasst:
Empfangen von Temperaturdaten und Herzfrequenzdaten der Person von einer Messeinheit in Form einer tragbaren Vorrichtung, die mindestens einen Sensor für die distale Hauttemperatur und einen Herzfrequenzsensor umfasst,
Bestimmen der Variabilität der distalen Hauttemperatur der Person basierend auf den Temperaturdaten im Laufe der Zeit und Bestimmen der Herzfrequenzvariabilität (HRV) der Person basierend auf den Herzfrequenzdaten,
Erfassen von Schlafphasen der Person während des Schlafs einer Person durch
i) Erfassen, dass sich die Person in einer Rapid Eye Movement(REM)-Schlafphase befindet, wenn die Variabilität der distalen Hauttemperatur unter einem ersten Grenzwert der Variabilität der distalen Hauttemperatur liegt und die Herzfrequenzvariabilität über einem ersten Grenzwert der Herzfrequenzvariabilität liegt, und
ii) Erfassen, dass sich die Person in einer Tiefschlafphase befindet, wenn die Variabilität der distalen Hauttemperatur unter einem zweiten Grenzwert der Variabilität der distalen Hauttemperatur liegt und die Herzfrequenzvariabilität unter einem zweiten Grenzwert der Herzfrequenzvariabilität liegt, und Bereitstellen von Informationen über die Qualität des Schlafs der Person basierend auf den erfassten Schlafphasen.

2. Verfahren nach Anspruch 1, wobei das Verfahren ferner Folgendes umfasst:
Schätzen eines optimalen Zeitpunkts zum Aufwachen und/oder eines optimalen Zeitpunkts zum Schlafengehen basierend auf den erfassten Schlafphasen.

3. Verfahren nach Anspruch 2, wobei das Verfahren Folgendes umfasst:
Bestimmen einer Länge eines Schlafzyklus der Person, und
Schätzen des optimalen Zeitpunkts zum Aufwachen basierend auf der Länge des Schlafzyklus, so dass die Person nach einem Nachtschlaf zum optimalen Zeitpunkt zum Aufwachen eine gewünschte Anzahl von Schlafzyklen geschlafen haben wird.

4. Verfahren nach Anspruch 2, wobei das Verfahren Folgendes umfasst:
Bestimmen einer Länge eines Schlafzyklus der Person,
Bestimmen eines festen Bezugspunkts innerhalb eines zirkadianen Rhythmus der Person,
Schätzen des optimalen Zeitpunkts zum Aufwachen basierend auf der Länge des Schlafzyklus und dem festen Bezugspunkt, so dass die Person zum optimalen Zeitpunkt zum Aufwachen eine gewünschte Anzahl von Schlafzyklen geschlafen haben wird.

5. Verfahren nach einem der Ansprüche 2 bis 4, wobei das Schätzen des optimalen Zeitpunkts zum Aufwachen oder des optimalen Zeitpunkts zum Schlafengehen ferner Folgendes umfasst:
Sammeln von Messdaten über eine Vielzahl von Tagen,
Berechnen einer Schätzung des zirkadianen Rhythmus der Person basierend auf den gesammelten Daten, und
Schätzen des optimalen Zeitpunkts zum Aufwachen und/oder des optimalen Zeitpunkts zum Schlafengehen basierend auf den erfassten Schlafphasen und der Schätzung des zirkadianen Rhythmus.

6. Verfahren nach einem der vorhergehenden Ansprüche, wobei das Bereitstellen der Informationen über die Qualität des Schlafs Folgendes umfasst:
Berechnen von Anteilen verschiedener Schlafphasen der Person basierend auf den erfassten Schlafphasen, und
Verwenden mindestens der berechneten Anteile als Informationen über die Schlafqualität.

7. Erfassungssystem, das eine computerimplementierte Erfassungseinheit mit einer Computervorrichtung oder einem Computersystem umfasst, wobei das Erfassungssystem für Folgendes konfiguriert ist:
Empfangen von ersten und zweiten Daten der Person von einer Messeinheit in Form einer tragbaren Vorrichtung, die mindestens einen Sensor für die distale Hauttemperatur und einen Herzfrequenzsensor umfasst, wobei die ersten Daten die distale Temperatur oder die Variabilität der distalen Temperatur darstellen und die zweiten Daten die Herzfrequenz oder die Herzfrequenzvariabilität darstellen,
Bestimmen der Variabilität der distalen Hauttemperatur der Person basierend auf den Temperaturdaten im Laufe der Zeit und Bestimmen der Herzfrequenzvariabilität (HRV) der Person basierend auf den Herzfrequenzdaten im Laufe der Zeit,
Erfassen der Schlafphasen der Person während des Schlafs einer Person durch
i) Erfassen, dass sich die Person in einer Rapid Eye Movement(REM)-Schlafphase befindet, wenn die Variabilität der distalen Hauttemperatur unter einem ersten Grenzwert der Variabilität der distalen Hauttemperatur liegt und die Herzfrequenzvariabilität über einem ersten Grenzwert der Herzfrequenzvariabilität liegt, und
ii) Erfassen, dass sich die Person in einer Tiefschlafphase befindet, wenn die Variabilität der distalen Hauttemperatur unter einem zweiten Grenzwert der Variabilität der distalen Hauttemperatur liegt und die Herzfrequenzvariabilität unter einem zweiten Grenzwert der Herzfrequenzvariabilität liegt, und
Bereitstellen von Informationen über die Qualität des Schlafs der Person basierend auf den erfassten Schlafphasen.

8. Erfassungssystem nach Anspruch 7, wobei das Erfassungssystem ferner für Folgendes konfiguriert ist:
Schätzen eines optimalen Zeitpunkts zum Aufwachen und/oder eines optimalen Zeitpunkts zum Schlafengehen basierend auf den erfassten Schlafphasen.

9. Erfassungssystem nach Anspruch 8, wobei das Schätzen des optimalen Zeitpunkts zum Aufwachen oder des optimalen Zeitpunkts zum Schlafengehen Folgendes umfasst:
Bestimmen einer Länge eines Schlafzyklus der Person, und
Schätzen des optimalen Zeitpunkts zum Aufwachen basierend auf der Länge des Schlafzyklus, so dass die Person nach einem Nachtschlaf zum optimalen Zeitpunkt zum Aufwachen eine gewünschte Anzahl von Schlafzyklen geschlafen haben wird.

10. Erfassungssystem nach einem der Ansprüche 8 oder 9, wobei das Schätzen des optimalen Zeitpunkts zum Aufwachen oder des optimalen Zeitpunkts zum Schlafengehen ferner Folgendes umfasst:
Sammeln von Messdaten über eine Vielzahl von Tagen,
Berechnen einer Schätzung des zirkadianen Rhythmus der Person basierend auf den gesammelten Daten, und
Schätzen des optimalen Zeitpunkts zum Aufwachen und/oder des optimalen Zeitpunkts zum Schlafengehen basierend auf den erfassten Schlafphasen und der Schätzung des zirkadianen Rhythmus.

11. Erfassungssystem nach einem der Ansprüche 7 bis 10, wobei das Erfassungssystem Folgendes umfasst:
eine Erfassungseinheit, die dazu konfiguriert ist, die Schritte des Empfangens der ersten Daten und der zweiten Daten, des Erfassens der Schlafphasen und des Bereitstellens der Informationen über die Schlafqualität durchzuführen, wobei die Erfassungseinheit eine tragbare Kommunikationsvorrichtung ist, die dazu konfiguriert ist, die ersten Daten und die zweiten Daten drahtlos von einer Messeinheit zu empfangen.

12. Erfassungssystem nach Anspruch 11, wobei das Erfassungssystem ferner eine Messeinheit in Form einer tragbaren Vorrichtung umfasst, beispielsweise einen Ring, einen Aktivitätstracker, eine Smartwatch, einen Ohrhörer, einen E-Sticker oder ein Band, und mindestens Folgendes umfasst:
einen Temperatursensor, der dazu konfiguriert ist, die distale Hauttemperatur zu messen,
einen Herzfrequenzsensor, und
eine drahtlose Kommunikationseinheit, und
wobei die Messeinheit dazu konfiguriert ist, die ersten Daten und die zweiten Daten zu bestimmen und die ersten Daten und die zweiten Daten über die drahtlose Kommunikationseinheit an die Erfassungseinheit zu senden.

13. Computerprogrammprodukt, das Anweisungen aufweist, die, wenn sie von einer Computervorrichtung ausgeführt werden, die Computervorrichtung zu Folgendem zu veranlassen:
Empfangen von ersten Daten und zweiten Daten der Person von einer Messeinheit in Form einer tragbaren Vorrichtung, die mindestens einen Sensor für die distale Hauttemperatur und einen Herzfrequenzsensor umfasst, wobei die ersten Daten die distale Temperatur oder die Variabilität der distalen Temperatur darstellen und die zweiten Daten die Herzfrequenz oder die Herzfrequenzvariabilität (HRV) darstellen,
Erfassen der Schlafphasen während des Schlafs einer Person durch
i) Erfassen, dass sich die Person in einer Rapid Eye Movement(REM)-Schlafphase befindet, wenn die Variabilität der distalen Hauttemperatur unter einem ersten Grenzwert der Variabilität der distalen Hauttemperatur liegt und die Herzfrequenzvariabilität über einem ersten Grenzwert der Herzfrequenzvariabilität liegt, und
ii) Erfassen, dass sich die Person in einer Tiefschlafphase befindet, wenn die Variabilität der distalen Hauttemperatur unter einem zweiten Grenzwert der Variabilität der distalen Hauttemperatur liegt und die Herzfrequenzvariabilität unter einem zweiten Grenzwert der Herzfrequenzvariabilität liegt, und
Bereitstellen der Informationen über die Qualität des Schlafs der Person basierend auf den erfassten Schlafphasen.

## Revendications

1. Procédé de détection des phases de sommeil d'une personne au moyen d'un système de détection comprenant une unité de détection mise en œuvre par ordinateur avec un dispositif ou un système informatique, le procédé comprenant les étapes consistant à
recevoir des données de température et de fréquence cardiaque de la personne à partir d'une unité de mesure sous la forme d'un dispositif portable comprenant au moins un capteur de température cutanée distale et un capteur de fréquence cardiaque,
déterminer la variabilité de la température cutanée distale de la personne en fonction des données de température dans le temps et déterminer la variabilité de la fréquence cardiaque (VFC) de la personne en fonction des données de fréquence cardiaque,
détecter des phases de sommeil de la personne dans le sommeil d'une personne en
i) détectant que la personne se trouve dans une phase de sommeil à mouvement oculaire rapide (REM) lorsque la variabilité de la température cutanée distale est inférieure à une première limite de variabilité de la température cutanée distale et que la variabilité de la fréquence cardiaque est supérieure à une première limite de variabilité de la fréquence cardiaque, et
ii) détectant que la personne est dans une phase de sommeil profond lorsque la variabilité de la température cutanée distale est inférieure à une seconde limite de variabilité de la température cutanée distale et que la variabilité de la fréquence cardiaque est inférieure à une seconde limite de variabilité de la fréquence cardiaque, et
fournir des informations sur la qualité du sommeil de la personne en fonction des phases détectés du sommeil.

2. Procédé selon la revendication 1, le procédé comprenant en outre
l'estimation d'un instant optimal pour un réveil et/ou d'un instant optimal pour s'endormir sur la base des phases de sommeil détectés.

3. Procédé selon la revendication 2, le procédé comprenant les étapes consistant à
déterminer la durée du cycle de sommeil de la personne, et
estimer l'instant de réveil optimal en fonction de la durée du cycle de sommeil de sorte qu'après une nuit de sommeil, la personne ait dormi pendant un nombre souhaité de cycles de sommeil à l'instant de réveil optimal.

4. Procédé selon la revendication 2, le procédé comprenant les étapes consistant à
déterminer la durée d'un cycle de sommeil de la personne,
déterminer un point de référence fixe à l'intérieur d'un rythme circadien de la personne,
estimer l'instant de réveil optimal sur la base de la durée du cycle de sommeil et du point de référence fixe de sorte que la personne ait dormi pendant un nombre souhaité de cycles de sommeil à l'instant de réveil optimal.

5. Procédé selon l'une quelconque des revendications 2 à 4, dans lequel l'estimation de l'instant optimal pour un réveil ou de l'instant optimal pour l'endormissement comprend en outre les étapes consistant à
collecter des données de mesure sur plusieurs jours,
calculer une estimation du rythme circadien de la personne en fonction des données recueillies, et
estimer l'instant optimal pour un réveil et/ou de l'instant optimal pour s'endormir sur la base des phases détectées du sommeil et de l'estimation du rythme circadien.

6. Procédé selon l'une quelconque des revendications précédentes, dans lequel la fourniture des informations sur la qualité du sommeil comprend les étapes consistant à
calculer les ratios des différentes phases de sommeil de la personne en fonction des phases détectées de sommeil, et
utiliser au moins les ratios calculés comme information sur la qualité du sommeil.

7. Système de détection comprenant une unité de détection mise en œuvre par ordinateur avec un dispositif ou système informatique de, dans lequel le système de détection est configuré pour
recevoir des premières données et des secondes données de la personne à partir d'une unité de mesure sous la forme d'un dispositif portable comprenant au moins un capteur de température cutanée distale et un capteur de fréquence cardiaque, les premières données représentant la température distale ou la variabilité distale de la température, et les secondes données représentant la fréquence cardiaque ou la variabilité de la fréquence cardiaque,
déterminer la variabilité de la température cutanée distale de la personne en fonction des données de température dans le temps et déterminer la variabilité de la fréquence cardiaque (VFC) de la personne en fonction des données de fréquence cardiaque dans le temps,
détecter les phases du sommeil de la personne dans le sommeil d'une personne en
i) détectant que la personne se trouve dans une phase de sommeil à mouvement oculaire rapide (REM) lorsque la variabilité de la température cutanée distale est inférieure à une première limite de variabilité de la température cutanée distale et que la variabilité de la fréquence cardiaque est supérieure à une première limite de variabilité de la fréquence cardiaque, et
ii) détectant que la personne est dans une phase de sommeil profond lorsque la variabilité de la température cutanée distale est inférieure à une seconde limite de variabilité de la température cutanée distale et que la variabilité de la fréquence cardiaque est inférieure à une seconde limite de variabilité de la fréquence cardiaque, et
fournir des informations sur la qualité du sommeil de la personne en fonction des phases de sommeil détectées.

8. Système de détection selon la revendication 7, dans lequel le système de détection est en outre configuré pour
estimer un instant optimal pour un réveil et/ou un instant optimal pour s'endormir en fonction des phases détectées du sommeil.

9. Système de détection selon la revendication 8, dans lequel l'estimation de l'instant optimal pour un réveil ou de l'instant optimal pour un endormissement comprend les étapes consistant à
déterminer la durée du cycle de sommeil de la personne, et
estimer l'instant de réveil optimal en fonction de la durée du cycle de sommeil de sorte qu'après une nuit de sommeil, la personne ait dormi un nombre souhaité de cycles de sommeil à l'instant de réveil optimal.

10. Système de détection selon l'une quelconque des revendications 8 ou 9, dans lequel l'estimation de l'instant optimal pour un réveil ou de l'instant optimal pour l'endormissement comprend en outre les étapes consistant à
collecter des données de mesure sur plusieurs jours,
calculer une estimation du rythme circadien de la personne en fonction des données recueillies, et
estimer l'instant optimal de réveil et/ou de l'instant optimal d'endormissement sur la base des phases de sommeil détectées et de l'estimation du rythme circadien.

11. Système de détection selon l'une quelconque des revendications 7 à 10, dans lequel le système de détection comprend
une unité de détection configurée pour effectuer les étapes de réception des premières données et des secondes données, de détection des étapes de sommeil, et de fourniture des informations sur la qualité du sommeil, l'unité de détection étant un dispositif de communication portable de configuré pour recevoir sans fil les premières données et les secondes données d'une unité de mesure.

12. Système de détection selon la revendication 11, dans lequel le système de détection comprend en outre une unité de mesure sous la forme d'un dispositif portable, tel qu'un anneau, un suiveur d'activité, une montre intelligente, un écouteur, un autocollant électronique ou une bande, comprenant au moins :
un capteur de température configuré pour mesurer la température distale cutanée,
un capteur de fréquence cardiaque, et
une unité de communication sans fil, et
l'unité de mesure étant configurée pour déterminer les premières données et les secondes données et envoyer les premières données et les secondes données à l'unité de détection par l'intermédiaire de l'unité de communication sans fil.

13. Produit de programme informatique comportant des instructions qui, lorsqu'elles sont exécutées par un dispositif informatique, amènent le dispositif informatique à
recevoir des premières données et des secondes données de la personne d'une unité de mesure sous la forme d'un dispositif portable comprenant au moins un capteur de température cutanée distale et un capteur de fréquence cardiaque, les premières données représentant la température distale ou la variabilité de température distale, et les secondes données représentant la fréquence cardiaque ou la variabilité de fréquence cardiaque (VFC),
détecter les phases de sommeil dans le sommeil d'une personne en
i) détectant que la personne se trouve dans une phase de sommeil à mouvement oculaire rapide (REM) lorsque la variabilité de la température cutanée distale est inférieure à une première limite de variabilité de la température cutanée distale et que la variabilité de la fréquence cardiaque est supérieure à une première limite de variabilité de la fréquence cardiaque, et
ii) détectant que la personne est dans une phase de sommeil profond lorsque la variabilité de la température cutanée distale est inférieure à une seconde limite de variabilité de la température cutanée distale et que la variabilité de la fréquence cardiaque est inférieure à une seconde variabilité de la fréquence cardiaque, et
fournir des informations sur la qualité du sommeil de la personne en fonction des phases de sommeil détectées.
